# FASCICULE DE BREVET EUROPEEN

(11) **EP 0 786 673 B1**
(45) Date de publication et mention de la délivrance du brevet: **27.09.2000**
(21) Numéro de dépôt: 97106066.0
(22) Date de dépôt: 30.07.1993
(51) Int. Cl.: G01V 3/22

(54) **Procédé et dispositif de sondage et de contrôle d'un volume de sous-sol**
Verfahren und Vorrichtung zum Abtasten und zum Untersuchen eines Untergrundvolumens
Method and apparatus for probing and inspecting an underground volume

(30) Priorité: 31.07.1992 FR 9209515; 28.12.1992 FR 9215759
(43) Date de publication de la demande: 30.07.1997
(62) Demande divisionnaire de: 93401981.1
(73) Titulaire: EUGESOL, F-92000 Nanterre (FR)
(72) Inventeur: Lantier, François, 38290 Villefontaine (FR); Frappin, Pierre, 38290 Villefontaine (FR)
(74) Mandataire: Dronne, Guy

(56) Documents cités:
- FR-A- 1 247 925
- US-A- 2 654 064
- US-A- 2 779 912
- US-A- 3 134 941

## Description

La présente invention a pour objet un procédé de sondage d'un volume de sous-sol destiné à permettre, par une opération simple, la connaissance de la nature et de la consistance des terrains.

Le problème de la nature du sous-sol se pose pour la construction d'ouvrages importants, le percement de tunnels et la recherche pétrolière, entre autres.

Dans le domaine de la recherche pétrolière, il est connu (depuis 1927, C. SCHLUMBERGER) de réaliser des diagraphies différées et, au moyen de celles-ci, d'obtenir la mesure de résistivité des formations traversées le long d'un sondage préalable à un forage. A cet effet, on utilise une sonde dite normale portant de quatre à six électrodes à l'aide desquelles est mesuré le log de résistivité.

Le principe de cette mesure consiste à injecter un courant continu dans le terrain, sous une tension de 200 à 600 volts avec une intensité de 200 à 400 mA, à mesurer la différence de potentiel induite entre deux électrodes de mesure, et à en déduire la résistivité locale, le résultat étant disponible en ohms.mètres. Mais, avec un tel système, l'investigation latérale est de l'ordre de 1 mètre autour du sondage. Il en résulte que, pour connaître la nature exacte du sous-sol, les forages doivent être multipliés, ce qui est long et, par suite, coûteux.

FR-A-1247 925 décrit un dispositif de sondage dans lequel trois électrodes d'émission de courant et quatre électrodes de mesure permettent d'obtenir trois mesures de la résistivité des terrains autour du forage. Mais ces informations, permettant d'établir un profilage, sont insuffisantes pour détecter la présence, à une distance supérieure à 1 mètre du forage, d'une hétérogénéité lithologique. Or, ce qui importe dans certaines applications, notamment dans les travaux publics, est non seulement la connaissance des couches traversées, mais également la connaissance de l'environnement du forage.

La présente invention a pour objet un procédé d'investigation d'un volume de sous-sol permettant de pallier cet inconvénient et de permettre en une seule opération la détermination de l'homogénéité de nature d'un volume de sous-sol important, permettant d'établir une cartographie du sous-sol. A titre d'exemple, la présente invention permet d'ausculter un volume cylindrique d'un diamètre de 1 à 100 mètres autour d'un trou de sondage, que ce trou soit vertical, horizontal ou incliné.

Un second objet de la présente invention est de permettre un contrôle de la qualité de l'injection effectuée de manière à répondre aux normes de sécurité.

Selon la présente invention, le procédé de sondage par mesure électrique du sol par application d'un courant au moyen de deux électrodes (A,B) et mesure de la tension développée entre deux électrodes de mesure est caractérisé en ce qu'il comporte les étapes suivantes :
a) on dispose dans un forage une sonde portant une pluralité d'électrodes d'émission (A) et une pluralité d'électrodes de mesure, le nombre d'électrodes d'émission étant supérieur au nombre d'électrodes de mesure, lesdites électrodes d'émission étant régulièrement reparties avec un pas fixe, lesdites électrodes de mesure étant intercalées entre les électrodes d'émission occupant une position centrale sur la sonde selon la longueur de celle-ci,
b) on dispose "à l'infini" une électrode fixe (B) d'émission;
c) on alimente successivement les électrodes d'émission et on mesure la tension entre un couple d'électrodes de mesure par quoi on obtient des informations représentatives du sol au niveau dudit couple d'électrodes et correspondant à des distances différentes dans le sol par rapport à ladite sonde, chaque distance étant associée à une électrode d'émission, et,
d) on répète l'étape c) pour des couples différents d'électrodes de mesure, par quoi on obtient des informations représentatives du sol correspondant à une pluralité de distances par rapport à la sonde pour une pluralité de niveaux.

De préférence, le courant d'injection est continu. La sonde selon l'invention utilisant la technique pôle-dipôle met en oeuvre, par exemple, trente-cinq électrodes disposées sur toute la longueur ou hauteur du forage (quinze, trente ou soixante mètres). La seconde électrode d'émission de courant B est reportée à l'infini (pratiquement quelques centaines de mètres) et l'électrode A est "mobile", les électrodes A étant successivement alimentées. Le nombre d'électrodes A est supérieur au nombre d'électrodes M de manière à pouvoir faire varier la distance A-Ma,Mb pour obtenir un sondage large autour de la sonde, Ma,Mb désignant un couple quelconque d'électrodes de mesure. En injectant un courant électrique sur les électrodes A, on obtient en appliquant la loi d'Ohm généralisée à un tripôle (A, Ma-Mb) une meure de résistivité apparente des terrains entre les deux électrodes de potentiel Ma-Mb fixes. Plus l'électrode A s'éloigne de Ma et Mb, plus la résistivité apparente affecte une épaisseur de terrains plus grande à partir de l'axe A, Ma,Mb, c'est-à-dire à partir de la sonde. Ce résultat résulte de la forme des lignes équipotentielles qui se développent. L'électrode active d'émission apparaît en quelque sorte mobile le long de la sonde, ce qui permet d'obtenir des volumes d'exploration importants étant donné que c'est la distance électrode d'injection-électrodes de potentiel qui détermine la profondeur des équipotentielles. Cette profondeur peut ainsi être importante de même que le nombre de points de mesure.

Selon une autre caractéristique de l'invention, le procédé de contrôle de la répartition d'une injection d'un matériau choisi parmi les bétons et les coulis dans le sol est caractérisé en ce qu'il comprend les étapes suivantes :
- avant l'injection, on dispose dans le forage une pluralité de sondes de mesure disposées parallèlement les unes aux autres et on effectue avec chaque sonde les étapes a) et d) de la revendication 1 par quoi on obtient une première série d'informations représentatives du sol
- on coule le béton dans ledit forage,
- on répète à l'aide desdites sondes les étapes a) et d) de la revendication 1 par quoi on obtient une deuxième série d'informations représentatives du sol après injection, et
- on compare lesdites informations pour chaque niveau et chaque distance desdites première et deuxième séries.

Elle est basée sur le fait que le béton ou le coulis à l'état liquide ou pâteux est un bon conducteur de l'électricité immédiatement après son injection alors qu'il devient très résistant après durcissement. On détecte les variations qui se sont produites entre l'état "0" avant injection et l'état "1" après injection pour contrôler le succès ou l'échec de l'injection.

L'invention concerne encore un dispositif de sondage par mesure électrique de la résistivité électrique du sol par application d'un courant électrique au moyen de deux électrodes d'émission (A, B) et mesure de la tension développée entre deux électrodes de mesure qui se caractérise en ce qu'il comprend :
- une sonde destinée à être mise en place dans un forage creusé dans le sol à sonder, ladite sonde comprenant une pluralité d'électrodes de mesure et une pluralité d'électrodes d'émission, le nombre d'électrodes d'émission étant supérieur au nombre d'électrodes de mesure, les électrodes de mesure étant intercalées entre les électrodes d'émission, lesdites électrodes d'émission étant disposées avec un pas fixe;
- une électrode d'émission disposée à "l'infini" par rapport au dit forage,
- une source d'énergie électrique,
- des moyens de mesure de tension électrique,
- des premiers moyens pour relier successivement chaque électrode d'émission à la source d'énergie électrique, et
- des deuxièmes moyens pour relier successivement chaque paire d'électrodes de mesure auxdits moyens de mesure de tension.

D'autres caractéristiques et avantages de l'invention apparaîtront au cours de la description qui va suivre d'un mode de réalisation particulier, donné à titre d'exemple non limitatif en regard des dessins qui représentent :
- la figure 1, un exemple de positionnement des électrodes d'injection et de potentiel ;
- la figure 2, un tableau des niveaux de mesures ;
- la figure 3, un exemple de réalisation d'une sonde ;
- la figure 4, deux exemples de résultat de mesures relevées dans un terrain particulier ;
- la figure 5, un exemple du résultat obtenu par un contrôle selon l'invention.

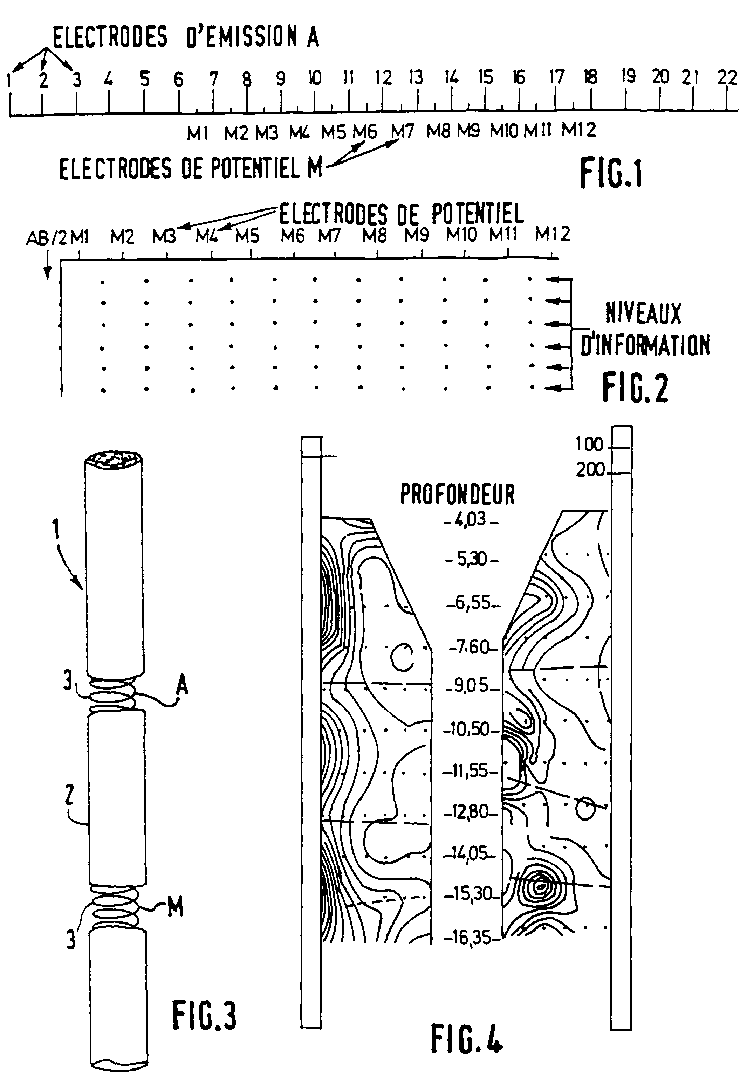

Sur la figure 1, on voit que l'électrode d'injection A peut prendre un grand nombre de positions, vingt-trois dans l'exemple représenté. Il s'agit en fait de vingt-trois électrodes qui sont successivement alimentées par un générateur émettant un courant de 10 à 300 mA sous une tension de 200 volts, par exemple. De préférence, ce courant est continu.

Le générateur est inclus dans un coffret comprenant un multiplexeur manuel et un milliampèremètre digital, le dispositif comprend également un coffret de réception incluant un multiplexeur automatique et un millivoltmètre digital. Une centrale d'acquisition et de gestion comprend un ordinateur PC 386 avec son programme de gestion des mesures et des moyens de mémorisation.

Comme schématisé sur la figure 1, les électrodes de potentiel M sont au nombre de douze et occupent les positions M1 à M12. Ces positions sont intercalées avec les vingt-trois positions de l'électrode d'injection A. En faisant varier les positions de A et des couples Ma-Mb, on obtient, comme représenté sur la figure 2, centre trente-deux mesures de résistivité avec six niveaux d'information disposés au droit des centres de symétrie de chaque coupe Ma-Mb en fonction de la demi-longueur AB et donc en fonction de la profondeur. Au droit de chaque couple Ma-Mb, on obtient deux mesures de résistivité par les deux positions symétriques de A par rapport à Ma-Mb.

L'écartement de deux électrodes M peut être quelconque, mais fixe pour un ensemble de mesures données. Les positions relatives de A sont alors parfaitement déterminées comme représenté sur la figure 1. Les électrodes sont incluses dans un câble et, pour assurer un bon contact des électrodes et du terrain, on remplit, avant les mesures, le trou de forage avec de l'eau ou de la boue.

Toute anomalie de résistivité est repérée et projetée, pour les résultats sur un rayon ainsi que son niveau. Or, les variations de résistivité indiquent des variations de structure du sous-sol. La résistivité est un bon indice de la qualité des terrains en matière de travaux souterrains puisque la plupart des accidents géologiques générateurs de difficultés lors de l'excavation et du soutènement sont argileux avec éventuellement présence d'eau. Ils se traduisent généralement par des résistivités faibles.

A contrario, les calcaires ont une résistivité plus élevée qui croît avec les roches primaires telles que le granit, par exemple. Le cylindre permet ainsi de déceler, dans un volume rocheux donné autour d'un forage, toute zone anormale dans un environnement a priori homogène et de la situer précisément en position par rapport aux électrodes de potentiel M. Mais, l'azimut autour du forage n'est bien entendu pas maîtrisé. On obtient ainsi une projection plane du cylindre électrique.

La figure 3 représente une partie d'une sonde selon l'invention. Elle se compose d'un câble isolé 1 comprenant, par exemple, quarante-huit conducteurs qui sont successivement dégagés de la gaine 2 pour former une boucle 3 qui est ainsi en contact avec le terrain. L'alimentation des boucles A d'injection et des boucles M est gérée respectivement par les multiplexeurs mentionnés précédemment. Bien entendu, le nombre d'électrodes d'émission et de mesure peut être quelconque, le nombre d'électrodes d'émission étant toujours supérieur au nombre d'électrodes de mesure.

La figure 4 représente le résultat obtenu au cours de deux forages de prospection. Ces forages peuvent être de faible section puisque la section de la sonde est elle-même très faible. Les deux schémas représentent des pseudo-sections qui rendent compte de toute anomalie détectée dans un cylindre entourant la sonde. Les lignes figurant à l'intérieur de chaque section sont des lignes d'isorésistivité, cette grandeur étant exprimée en ohms.mètres.

En haut de la ligne représentant le forage, on trouve de la terre végétale sur une hauteur de l'ordre de 1 mètre puis, dans l'exemple représenté, des couches de calcaire gris bleuté légèrement argileux. Sur la figure de gauche, on voit que les lignes d'isorésistivité sont concentrées autour du trou de forage. Il s'agit, et cela a été corroboré par un carottage, d'une couche dense de calcaire massif gris bleuté légèrement argileux. Mais, dès que l'on s'éloigne de l'axe du forage, les lignes deviennent plus espacées, ce qui traduit une résistivité plus faible. Il s'agit de couches d'argile plus ou moins déconsolidée avec quelques passages de blocs de calcaire. La figure de droite représente les résultats d'un forage qui a été effectué au voisinage du premier forage et qui confirme l'existence de couches d'argile.

Le procédé selon l'invention rend compte de tout accident géologique se produisant dans un rayon donné autour du forage de sondage, mais sans indiquer la position exacte de cet accident.

Les applications du procédé sont immédiates : reconnaissance autour de forages verticaux. L'investigation latérale est fonction de la profondeur réelle de l'ouvrage et peut être augmentée ; reconnaissance à l'avancement dans des forages horizontaux, notamment dans le cas d'ouvrages souterrains avec tunnelier et elle permet de repérer les karsts ou dolines ainsi que les hétérogénéités lithologiques. Il peut également être mis en oeuvre dans les forages pétroliers pour repérer les structures et dans les opérations préalables à la fracturation hydraulique des roches.

La présente invention vise également un procédé électrique et un dispositif de contrôle d'injections de coulis ou de béton dans le sol. Dans une telle application, la nature du sous-sol au voisinage de l'ouvrage est connue et c'est pour remédier à des faiblesses détectées que l'on injecte du béton.

De telles injections sont courantes quand il s'agit de réaliser une consolidation dans le sol en vue d'implanter un ouvrage d'art tel qu'un tunnel lorsque le sol présente une structure poreuse et insuffisamment résistante. Dans ces conditions, on fore un trou et l'on injecte un coulis de béton qui se répand dans le sol. Elles sont aussi réalisées, lorsqu'il s'agit d'effectuer une consolidation, autour de galeries et tunnels, au sein de massifs rocheux, dans des formations meubles (par exemple du sable), dans des remblais ou dans les carrières. Malheureusement, compte tenu de l'hétérogénéité des sols, le coulis peut se diriger dans des zones non désirées et laisser creuses les zones intéressant l'implantation de l'ouvrage. Il est également souhaitable de vérifier l'injection réalisée dans les voiles de béton utilisés pour les barrages et dans les têtes de tunnel.

A partir d'un état "0", après injection d'un coulis de béton dans le sol, on procède à une seconde série de mesures de l'état "1". Ces mesures sont effectuées moins de trois semaines après l'injection du coulis pour que celui-ci soit encore bon conducteur électrique, sa conductibilité s'atténuant au fur et à mesure de sa prise.

Des mesures strictement identiques à la mesure effectuée dans l'état "0" sont alors réalisées avec le même nombre de sondes, la même disposition géométrique et la même chaîne de mesure.

On compare ensuite les six niveaux d'information état "1" aux six niveaux état "0", le rapport obtenu mesurant les variations de résistivité. On obtient ainsi par contraste une représentation graphique des variations de résistivité entre les deux états. Les valeurs de résistivité mesurées dans l'état "1" sont en général plus faibles que celles mesurées dans l'état "0" car les coulis sont en général de 5 à 50 fois plus conducteurs que les terrains.

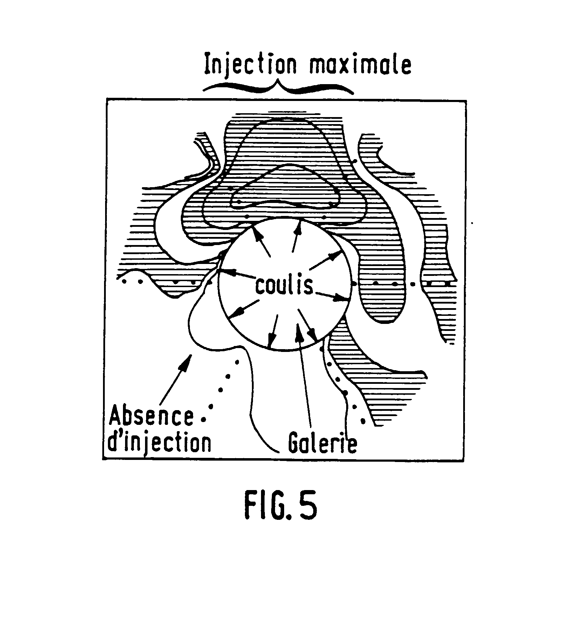

La figure 5 montre la répartition d'un coulis derrière le parement d'une galerie avec en radier une absence d'injection se traduisant par un rapport 1 entre les deux états.

## Revendications

1. Procédé de sondage par mesure électrique de la résistivité du sol par application d'un courant électrique au moyen de deux électrodes d'émission (A,B) et mesure de la tension développée entre deux électrodes de mesure (M) caractérisé en ce qu'il comprend les étapes suivantes :
a) on dispose dans un forage une sonde portant une pluralité d'électrodes d'émission (A) et une pluralité d'électrodes de mesure, le nombre d'électrodes d'émission étant supérieur au nombre d'électrodes de mesure, lesdites électrodes d'émission étant régulièrement reparties avec un pas fixe, lesdites électrodes de mesure étant intercalées entre les électrodes d'émission occupant une position centrale sur la sonde selon la longueur de celle-ci,
b) on dispose "à l'infini" une électrode fixe (B) d'émission;
c) on alimente successivement les électrodes d'émission et on mesure la tension entre un couple d'électrodes de mesure par quoi on obtient des informations représentatives du sol au niveau dudit couple d'électrodes et correspondant à des distances différentes dans le sol par rapport à ladite sonde, chaque distance étant associée à une électrode d'émission, et,
d) on répète l'étape c) pour des couples différents d'électrodes de mesure, par quoi on obtient des informations représentatives du sol correspondant à une pluralité de distances par rapport à la sonde pour une pluralité de niveaux.

2. Procédé selon la revendication 1 caractérisé en ce que le courant électrique est continu.

3. Procédé de sondage selon l'une quelconque des revendications 1 et 2 caractérisé en ce que pour chaque couple d'électrodes de mesure on associe les mesures faites en relation avec deux électrodes d'émission disposées symétriquement sur la sonde par rapport au dit couple d'électrodes de mesure.

4. Procédé de sondage selon l'une quelconque des revendications 1 à 3, caractérisé en ce qu'on alimente les électrodes d'émission avec un courant dont l'intensité est comprise 10 et 300 mA et sous une tension de 200 volts.

5. Application du procédé selon l'une quelconque des revendications 1 à 4 au contrôle de la répartition d'une injection d'un matériau choisi dans le groupe comprenant les bétons et les coulis dans le sol caractérisé en ce qu'il comprend les étapes suivantes :
- avant l'injection, on dispose dans le forage une pluralité de sondes de mesure disposées parallèlement les unes aux autres et on effectue avec chaque sonde les étapes a) et d) de la revendication 1 par quoi on obtient une première série d'informations représentatives du sol avant injection,
- on coule le béton dans ledit forage,
- on répète à l'aide desdites sondes les étapes a) et d) de la revendication 1 par quoi on obtient une deuxième série d'informations représentatives du sol après injection, et
- on compare lesdites informations pour chaque niveau et chaque distance desdites première et deuxième séries.

6. Sonde pour la mise en oeuvre du procédé selon l'une quelconque des revendications 1 à 5, caractérisée en ce qu'elle est constituée par un câble isolé gainé renfermant intérieurement une pluralité de conducteurs électriques, lesdits conducteurs électriques étant successivement dégagés de ladite gaine isolante selon la longueur dudit câble pour former des boucles en contact avec le terrain lesdites boucles constituant lesdites électrodes de mesure et d'émission.

7. Dispositif de sondage par mesure électrique de la résistivité électrique du sol par application d'un courant électrique au moyen de deux électrodes d'émission (A, B) et mesure de la tension développée entre deux électrodes de mesure caractérisé en ce qu'il comprend :
- une sonde destinée à être mise en place dans un forage creusé dans le sol à sonder, ladite sonde comprenant une pluralité d'électrodes de mesure et une pluralité d'électrodes d'émission le nombre d'électrodes d'émission étant supérieur au nombre d'électrodes de mesure, les électrodes de mesure étant intercalées entre les électrodes d'émission, lesdites électrodes d'émission étant disposées avec un pas fixe ;
- une électrode d'émission disposée à "l'infini" par rapport au dit forage,
- une source d'énergie électrique,
- des moyens de mesure de tension électrique,
- des premiers moyens pour relier successivement chaque électrode d'émission à la source d'énergie électrique, et
- des deuxièmes moyens pour relier successivement chaque paire d'électrodes de mesure aux dits moyens de mesure de tension.

8. Dispositif de sondage selon la revendication 7 caractérisé en ce que la source de courant électrique est continue.

## Claims

1. Method for test-boring by electrical measurement of the resistivity of the soil, by applying an electrical current by means of two transmission electrodes (A, B) and measuring the voltage developed between two measurement electrodes (M), characterised in that it comprises the following steps:
a) a probe is arranged inside a drill-hole, which probe carries a plurality of transmission electrodes (A) and a plurality of measurement electrodes, the number of transmission electrodes being greater than the number of measurement electrodes, said transmission electrodes being distributed at regular intervals with a fixed pitch, said measurement electrodes being interpolated between the transmission electrodes occupying a central position on the probe, along the length thereof,
b) a fixed transmission electrode (B) is arranged "at infinity";
c) power is supplied successively to the transmission electrodes and the voltage between a pair of measurement electrodes is measured, by which information is obtained representative of the soil at the level of said pair of electrodes and corresponding to different distances in the soil relative to said probe, each distance being associated with a transmission electrode, and
d) step c) is repeated for different pairs of measurement electrodes, by which information is obtained representing the soil corresponding to a plurality of distances relative to the probe for a plurality of levels.

2. Method according to Claim 1 characterised in that the electrical current is direct.

3. Test-boring method according to any one of Claims 1 and 2 characterised in that for each pair of measurement electrodes an association is made between the measurements made in relation with two transmission electrodes arranged symmetrically on the probe relative to said pair of measurement electrodes.

4. Test-boring method according to any one of Claims 1 to 3, characterised in that the transmission electrodes are supplied with a current the intensity of which is between 10 and 300 mA and at a voltage of 200 volts.

5. Application of the method according to any one of Claims 1 to 4 to the checking of the distribution of an injection into the soil of a material chosen from among the group comprising types of concrete and slurry, characterised in that it comprises the following steps:
- before injection, a plurality of measurement probes, arranged parallel to each other, is placed inside the drill-hole and steps a) and d) of Claim 1 are effected with each probe, which produces a first series of pieces of information representing the soil before injection;
- the concrete is cast in said drill-hole,
- steps a) and d) of Claim 1 are repeated, which produces a second series of pieces of information representing the soil after injection, and
- said information is compared for each level and each distance of said first and second series.

6. Probe for implementing the method according to any one of Claims 1 to 5, characterised in that it is constituted by a sheathed insulated cable enclosing inside it a plurality of electrical conductors, said electrical conductors being cleared successively from said insulating sheath along the length of said cable in order to form loops in contact with the earth, said loops constituting said measurement and transmission electrodes.

7. Device for test-boring by electrical measurement of the electrical resistivity of the soil, by applying an electrical current by means of two transmission electrodes (A, B) and measuring the voltage developed between two measurement electrodes, characterised in that it comprises:
- a probe intended to be placed inside a drill-hole made in the soil to be test-bored, said probe comprising a plurality of measurement electrodes and a plurality of transmission electrodes, the number of transmission electrodes being greater than the number of measurement electrodes, the measurement electrodes being interpolated between the transmission electrodes, said transmission electrodes being arranged at a fixed pitch,
- a transmission electrode arranged at "infinity" relative to said drill-hole,
- a source of electrical power,
- means for measuring electrical voltage,
- first means for successively connecting each transmission electrode to the source of electrical power, and
- second means for successively connecting each pair of measurement electrodes to said means for measuring voltage.

8. Test-boring device according to Claim 7 characterised in that the source of electrical current is direct.

## Patentansprüche

1. Verfahren zum Untersuchen durch elektrische Messung des Bodenwiderstands durch Anlegen eines elektrischen Stroms mittels zweier Emissionselektroden (A, B) und Messen der zwischen zwei Messelektroden (M) ausgebildeten Spannung, dadurch gekennzeichnet, dass es die folgenden Schritte umfasst:
a) Anordnen einer Sonde, die eine Mehrzahl von Emissionselektroden (A) und eine Mehrzahl von Messelektroden trägt, in einer Bohrung, wobei die Anzahl an Emissionselektroden größer als die Anzahl an Messelektroden ist, die Emissionselektroden mit fixem Abstand gleichmäßig verteilt sind und die Messelektroden zwischen den Emissionselektroden eingefügt sind und eine zentrale Position an der Sonde entsprechend der Länge derselben einnehmen,
b) Anordnen einer fixen Emissionselektrode (B) "im Unendlichen",
c) sukzessives Speisen der Emissionselektroden und Messen der Spannung zwischen einem Messelektrodenpaar, wodurch für den Boden in Höhe des Elektrodenpaars repräsentative Informationen erhalten werden, die unterschiedlichen Entfernungen im Boden in Bezug auf die Sonde entsprechen, wobei jede Entfernung einer Emissionselektrode zugeordnet ist, und
d) Wiederholen des Schritts c) für verschiedene Messelektrodenpaare, wodurch für den Boden repräsentative Informationen für eine Mehrzahl von Höhenlagen erhalten werden, die einer Mehrzahl von Entfernungen in Bezug auf die Sonde entsprechen.

2. Verfahren nach Anspruch 1, dadurch gekennzeichnet, dass der elektrische Strom Gleichstrom ist.

3. Verfahren zum Untersuchen nach einem der Ansprüche 1 und 2, dadurch gekennzeichnet, dass für jedes Messelektrodenpaar die Messungen vereinigt werden, die in Relation zu zwei Emissionselektroden durchgeführt wurden, welche in Bezug auf das Messelektrodenpaar symmetrisch an der Sonde angeordnet sind.

4. Verfahren zum Untersuchen nach einem der Ansprüche 1 bis 3, dadurch gekennzeichnet, dass die Emissionselektroden mit einem Strom einer Stärke von 10 bis 300 mA und unter einer Spannung von 200 V gespeist werden.

5. Anwendung des Verfahrens nach einem der Ansprüche 1 bis 4 zum Überwachen der Verteilung einer Injektion eines Materials, ausgewählt aus der Gruppe bestehend aus Beton und Ausfugmasse, im Boden, dadurch gekennzeichnet, dass es die folgenden Schritte umfasst:
- Anordnen einer Mehrzahl von parallel zueinander angeordneten Messsonden in der Bohrung und Durchführen der Schritte a) und d) des Anspruchs 1 mit jeder Sonde vor der Injektion, wodurch eine erste Serie von für den Boden vor der Injektion repräsentativen Informationen erhalten wird,
- Gießen des Betons in die Bohrung,
- Wiederholen der Schritte a) und d) des Anspruchs 1 mit Hilfe der Sonden, wodurch eine zweite Serie von für den Boden nach der Injektion repräsentativen Informationen erhalten wird, und
- Vergleichen der Informationen der ersten und zweiten Serie für jede Höhenlage und jede Entfernung.

6. Sonde zur Durchführung des Verfahrens nach einem der Ansprüche 1 bis 5, dadurch gekennzeichnet, dass sie durch ein isolierend umhülltes Kabel gebildet ist, das im Inneren eine Mehrzahl von elektrischen Leitern enthält, welche elektrischen Leiter je nach Länge des Kabels nach und nach aus der Isolierhülle freigegeben werden, um in Kontakt mit dem Gelände befindliche Leitungsschleifen zu bilden, welche Leitungsschleifen die Mess- und Emissionselektroden bilden.

7. Vorrichtung zur Untersuchung durch elektrische Messung des elektrischen Bodenwiderstands durch Anlegen eines elektrischen Stroms mittels zweier Emissionselektroden (A, B) und Messen der zwischen zwei Messelektroden ausgebildeten Spannung, dadurch gekennzeichnet, dass sie folgendes umfasst:
- eine Sonde zur Anordnung in einer in dem zu untersuchenden Boden ausgehobenen Bohrung, welche Sonde eine Mehrzahl von Messelektroden und eine Mehrzahl von Emissionselektroden aufweist, wobei die Anzahl an Emissionselektroden größer als die Anzahl an Messelektroden ist, die Messelektroden zwischen den Emissionselektroden eingefügt sind und die Emissionselektroden mit fixem Abstand angeordnet sind,
- eine in Bezug auf die Bohrung "im Unendlichen" angeordnete Emissionselektrode,
- eine elektrische Energiequelle,
- Einrichtungen zur Messung der elektrischen Spannung,
- erste Einrichtungen zum sukzessiven Anschließen jeder Emissionselektrode an die elektrische Energiequelle und
- zweite Einrichtungen zum sukzessiven Anschließen jedes Messelektrodenpaars an die Einrichtungen zur Spannungsmessung.

8. Vorrichtung zur Untersuchung nach Anspruch 7, dadurch gekennzeichnet, dass die elektrische Stromquelle eine Gleichstromquelle ist.
